# EUROPÄISCHE PATENTSCHRIFT

(11) **EP 1 629 789 B1**
(45) Veröffentlichungstag und Bekanntmachung des Hinweises auf die Patenterteilung: **16.05.2007**
(21) Anmeldenummer: 04020625.2
(22) Anmeldetag: 31.08.2004
(51) Int. Cl.: A61B 19/00

(54) **Fluoroskopiebild-Verifizierung**
Fluoroscopic image verification
Vérification d'image fluoroscopique

(43) Veröffentlichungstag der Anmeldung: 01.03.2006
(73) Patentinhaber: BrainLAB AG, 85551 Kirchheim/Heimstetten (DE)
(72) Erfinder: Kraus, Florian, 85540 Haar (DE); Maier, Christian, 81827 München (DE)
(74) Vertreter: Schwabe - Sandmair - Marx

(56) Entgegenhaltungen:
- US-A- 5 951 475
- US-A1- 2004 015 176
- US-B2- 6 662 036
- US-B2- 6 697 664

## Beschreibung

Die vorliegende Erfindung betrifft ein Verfahren zur Verifizierung der Registrierung eines Fluoroskopiebildes. Insbesondere betrifft die Erfindung die Verifizierung der Akquirierungsgenauigkeit eines Fluoroskopiebildes, das innerhalb einer fluoroskopiebasierten Navigationssoftware registriert wird.

Fluoroskopiebilder werden beispielsweise mit Hilfe eine C-Bogen-Flouroskopiegerätes akquiriert und können an ein Navigationssystem übergeben werden, welches diese Bilder als Basis für die bildunterstützte bzw. bildgeführte Chirurgie verwendet. Eine Voraussetzung für die genaue bildgeführte bzw. bildunterstützte Chirurgie liegt darin, eine räumliche Korrelation zwischen der virtuellen Darstellung der Patienten- oder Körperdaten mit der tatsächlichen Anatomie des Patienten herzustellen. Dieses Verfahren wird Registrierung genannt. Die vorliegende Erfindung gestattet es, die Genauigkeit dieses Registrierungsprozesses für Fluoroskopiebilder zu verifizieren.

Notwendig wird dies, weil bei dem genannten Registrierungsverfahren Fehler auftreten können. Oftmals wird die Registrierung dadurch erzielt, dass sich Markierungen auf den Fluoroskopiebildern abbilden, welche in bekannter Position an einem Aufsatz der C-Bogen-Strahlungsquelle angeordnet sind. Falls sich dabei aber nicht eine ausreichende Anzahl von Markern abbildet, oder die Abbildung ungenau ist, kann es zu Registrierungsfehlern kommen.

Die US-Patente Nr. 6,697,664; 6,675,040 und 6,662,036 enthalten Beschreibungen über das Prinzip der Fluoroskopie-Navigation, geben jedoch keine Lösungen für die oben aufgezeigten Probleme.

Es ist die Aufgabe der vorliegenden Erfindung, ein Verfahren zur Verifizierung der Registrierung eines Fluoroskopiebildes bereitzustellen, welches eine verlässliche und genaue Navigation und bildunterstützte bzw. bildgeführte Chirurgie ermöglicht. Diese Aufgabe wird durch ein Verfahren gemäß dem Anspruch 1 gelöst.

Ferner stellt die Erfindung noch ein Vorrichtungssystem zur Verifizierung der Registrierung eines Fluoroskopiebildes gemäß dem Anspruch 4 zur Verfügung, und sie betrifft weiterhin ein Programm, das, wenn es auf einem Computer läuft oder in einem Computer geladen ist, den Computer veranlasst, ein oben genannten Verfahren durchzuführen bzw. ein Computerprogramm-Speichermedium, das ein solches Programm aufweist. Die Unteransprüche definieren bevorzugte Ausführungsformen der Erfindung.

Die mit der Erfindung erzielbaren Vorteile beruhen, was das Verfahren betrifft, auf der Durchführung der folgenden Schritte:
- mit Hilfe eines Fluoroskopiegerätes wird eine Fluoroskopieaufnahme erstellt;
- die Fluoroskopieaufnahme wird mittels auf ihr abgebildeter Markierungspunkte auf vorakquirierte Körperbilddaten eines computergestützten, medizinischen Navigationssystems registriert, wobei die Markierungspunkte Abbilder von Markierungen an der Strahlungsquelle sind;
- eine künstliche Landmarkenanordnung, die im Navigationssystem geortet und verfolgt werden kann, wird in den Strahlengang des Fluoroskopiegerätes eingebracht, und ihre Raumposition zum Zeitpunkt der Erstellung der Fluoroskopieaufnahme wird erfasst und gespeichert, und
- die Übereinstimmung des Bildschattens der künstlichen Landmarkenanordnung mit der im Navigationssystem wiedergegebenen Position dieser Landmarkenanordnung zum Zeitpunkt der Erstellung der Aufnahme wird mittels der Ausgabeeinheit des Navigationssystems geprüft.

Die Verwendung der künstlichen Landmarkenanordnung und die Prüfung der Übereinstimmung ihres Bildschattens mit der im Navigationssystem wiedergegebenen Position ermöglicht eine Verifizierung der Registrierungsgenauigkeit unabhängig von der ansonsten verwendeten Patienten-Registrierungs- und Tracking-Ausstattung. Dabei ist es im Rahmen der Erfindung nicht notwendig, dass die Position der Landmarkenanordnung dieselbe bleibt, es kann also darauf verzichtet werden, die Landmarkenanordnung mit Hilfe komplizierter Gerätschaften zu befestigen, die bei der gesamten Tätigkeit im Wege wären. Die Erfindung gestattet wegen der Speicherung der Raumposition der Landmarkenanordnung auch deren Verschiebung oder Entfernung nach der Bilderstellung. Dies macht es beispielsweise möglich, die künstliche Landmarkenanordnung einfach auf den Patienten aufzulegen, während die Verifizierungsaufnahme erstellt wird, da Verschiebungen der Haut oder der Abdeckung des Patienten und damit einhergehende Verschiebungen der Landmarkenanordnung die Verifizierung nicht negativ beeinflussen. Es ist somit auch nicht mehr nötig, die Landmarkenanordnung fest an einem freigelegten Knochen anzuordnen, so dass die Erfindung einen minimal invasiven oder sogar nichtinvasiven Weg zur Verifizierung der Registrierungsgenauigkeit eines Fluoroskopiebildes bietet.

Ein zusätzlicher Vorteil besteht noch darin, dass die Verwendung der künstlichen Landmarkenanordnung eine Fluoroskopiebild-Verifizierung auch dann gestattet, wenn eine herkömmliche Verifizierung oder Registrierung mit Hilfe von natürlichen Landmarken nicht möglich ist oder nur möglich wäre, wenn weitere invasive Eingriffe gemacht würden. Dies gilt beispielsweise für die Registrierung in Weichteilen oder an langen Knochen im Zwischenbereich des Knochens, wo sinnvoll keine natürlichen Landmarken akquiriert werden können.

Wenn bei dem erfindungsgemäßen Verfahren eine mangelnde Übereinstimmung festgestellt wird, kann erfindungsgemäß die Erstellung einer neuen Fluoroskopieaufnahme angefordert oder ausgelöst werden. Wie schon oben erwähnt, ist es im Rahmen der vorliegenden Erfindung von Vorteil, wenn die künstliche Landmarkenanordnung wieder entfernbar in den Strahlengang des Fluoroskopiegeräts eingebracht wird. Die weitere Navigation (bildunterstützte Chirurgie) kann mit Hilfe einer gesondert bereitgestellten Navigationsreferenz (Referenzstern) durchgeführt werden.

Das erfindungsgemäße Vorrichtungssystem zur Verifizierung der Registrierung eines Fluoroskopiebildes, das mit Hilfe eines Fluoroskopiegeräts erstellt wurde, weist auf:
- Markierungen an der Strahlungsquelle des Fluoroskopiegerätes, die auf einer Fluoroskopieaufnahme als Markierungspunkt-Abbilder abgebildet werden;
- ein computergestütztes, medizinisches Navigationssystems, mittels dem die Fluoroskopieaufnahme auf vorakquirierte Körperbilddaten registriert wird; gekennzeichnet durch
- eine vom Navigationssystem ortbare und verfolgbare, künstliche Landmarkenanordnung, die bei der Erstellung der Fluoroskopieaufnahme in den Strahlengang des Fluoroskopiegerätes einbringbar ist; durch
- eine Ausgabeeinheit des Navigationssystems zur Überprüfung der Übereinstimmung des Bildschattens der künstlichen Landmarkenanordnung mit der im Navigationssystem wiedergegebenen Position dieser Landmarkenanordnung; und durch
- eine Speichereinrichtung, welche die Raumposition der Landmarkenanordnung bei der Erstellung der Fluoroskopieaufnahme durch das Navigationssystem erfasst und speichert.

Die Speichereinrichtung kann Teil der Computereinheit des Navigationssystems sein. Dies ist sinnvoll, weil das Navigationssystem selbst auch die Position der Landmarkenanordnung mit Hilfe eines Trackingsystems orten bzw. verfolgen wird. In diesem Zusammenhang wird klar, dass gemäß einer bevorzugten Ausführungsform der Erfindung die künstliche Landmarkenanordnung Referenzmarker aufweisen kann, die an einer Haltestruktur angeordnet sind und von der Tracking- bzw. Kameraeinheit des Navigationssystems positionell bestimmt und verfolgt werden können, wobei die Landmarkenanordnung insbesondere im Navigationssystem vorkalibriert bzw. der Geometrie nach bekannt ist.

Die Haltestruktur besteht vorzugsweise aus einem mittels Röntgenstrahlung abbildbaren, insbesondere für Röntgenstrahlung teil- oder undurchlässigem Material, oder sie weist ein solches Material auf. Bei einer bevorzugten Ausführungsform ist die Haltestruktur kreuzförmig mit Referenzmarkern an den Außenenden ausgebildet.

Die Erfindung wird im Weiteren anhand einer Ausführungsform näher erläutert. Die Erfindung kann alle hierin beschriebenen Merkmale einzeln oder in jedweder Kombination umfassen, und die einzige beiliegende Figur, auf die nunmehr Bezug genommen wird, zeigt schematisch ein erfindungsgemäßes Vorrichtungssystem.

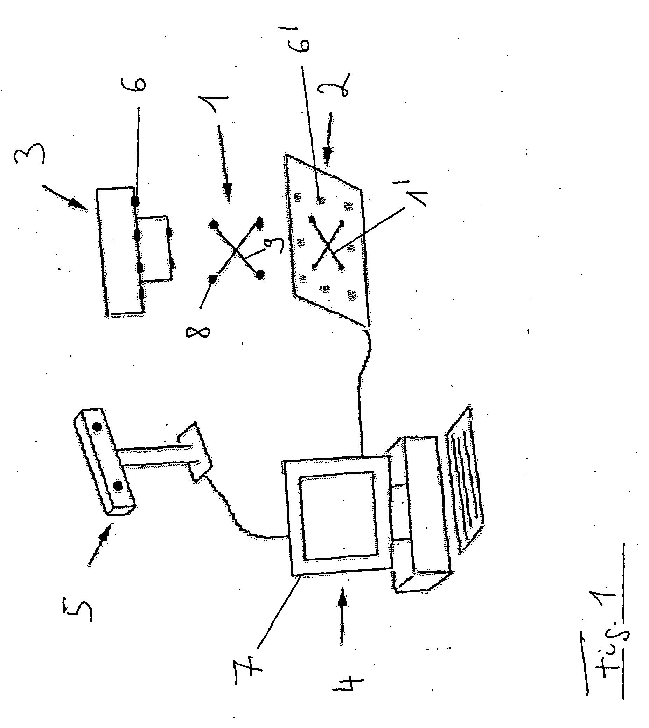

In der Figur 1 ist schematisch ein chirurgischen Navigationssystem dargestellt, dessen Computereinheit das Bezugszeigen 4 und dessen Kamera- bzw. Trackingeinheit das Bezugszeichen 5 trägt. Das Navigationssystem weist eine Bildschirmausgabe 7 auf.

Mit dem Navigationssystem wird die Fluoroskopieeinrichtung überwacht, von der in Figur 1 nur ein Aufsatz 3 dargestellt ist, der an der Strahlungsquelle des nicht gezeigten Fluoroskopiegeräts angebracht ist. Der Aufsatz 3 weist Markierungen 6 auf, die sich bei der Erstellung einer Fluoroskopieaufnahme 2 auf dieser als Markierungen 6' abbilden. Durch eine im Navigationsteam bekannte und vorkalibrierte Anordnung verschiedener Markierungen 6, auch auf verschiedenen Ebenen, lässt sich die dargestellte Fluoroskopieaufnahme zunächst schon prinzipiell im Navigationssystem registrieren, das heißt ihr Bildinhalt kann mit den Daten, die dem Navigationssystem zur Verfügung stehen, in räumliche Korrelation gebracht werden. Dazu wird vorteilhafter Weise auch die räumliche Position des nicht dargestellten Fluoroskopiegerätes im Navigationssystem bestimmt, wenn die Aufnahme ausgelöst wird.

Ferner ist in Figur 1 noch die künstliche Landmarkenanordnung 1 dargestellt, die im Strahlengang zwischen dem Aufsatz 3 und der Aufnahme 2 liegt. Sie weist eine kreuzförmige Haltestruktur 9 auf, an deren Enden Marker bzw. Referenzmarker 8 angebracht sind, welche durch das Navigationssystem, insbesondere durch die Kameraeinrichtung 5 positionell bestimmt und verfolgt werden können.

Die künstliche Landmarkenanordnung 1 wird als 1' auf der Fluoroskopieaufnahme 2 abgebildet. Die Anordnung der Referenzmarker 8 an der Landmarkenanordnung 1 wird durch das stereotaktische chirurgische System bzw. das Navigationssystem 4, 5 eindeutig wiedererkannt; vorteilhafterweise stellt sie eine eindeutig identifizierbare Anordnung dar, deren Geometrie im Navigationssystem hinterlegt ist.

Wie oben schon angedeutet, wird mittels der Markierungen 6 eine räumliche Relation zwischen der Fluoroskopieaufnahme 2 und dem Aufsatz 3 (und damit dem Fluoroskopiegerät) bestimmt. Diese räumliche Relation wird verwendet, um die Landmarkenanordnung 1 virtuell darzustellen.

Um nun die Genauigkeit der räumlichen Relation zwischen der Aufnahme 2 und dem Aufsatz 3 zu verifizieren, wird die Landmarkenanordnung 1 auf der Aufnahme 2 abgebildet und eine virtuelle Darstellung der Landmarkenanordnung 1 wird mittels des Navigationssystems und auf der Bildschirmausgabe 7 dem Abbild der Anordnung 1 auf der Aufnahme 2 überlagert. Ein Vergleich der beiden Darstellungen gestattet eine Genauigkeitsprüfung, das heißt, wenn das Abbild der Anordnung 1 mit seinem virtuellen Bild aus dem Navigationssystem übereinstimmt, ist die Aufnahme ausreichend genau. Wenn dies nicht der Fall ist, muss die Aufnahme wiederholt werden.

Darüber hinaus wird eine Bewegung der Landmarkenanordnung 1 bezüglich des Aufsatzes 3, die nach der Bilderstellung erfolgt, die Verifizierung nicht beeinträchtigen, da die Raumposition der Landmarkenanordnung zum Zeitpunkt der Bilderstellung durch das Navigationssystem erkannt (mittels der Referenzmarker 8, die durch die Trackingeinrichtung 5 verfolgt werden) und gespeichert wird. Auf diese Weise muss die Landmarkenanordnung 1 bei der Bildakquirierung und der Bildverifizierung nicht unbedingt in derselben Position sein; sie kann nach der Verifizierung entfernt werden. Die weitere Navigation (bildunterstützte Chirurgie) kann mit Hilfe einer gesondert bereitgestellten Navigationsreferenz (Referenzstern) durchgeführt werden.

## Patentansprüche

1. Verfahren zur Verifizierung der Registrierung eines Fluoroskopiebildes mit den folgenden Schritten:
- mit Hilfe eines Fluoroskopiegerätes wird eine Fluoroskopieaufnahme erstellt;
- die Fluoroskopieaufnahme (2) wird mittels auf ihr abgebildeter Markierungspunkte (6') auf vorakquirierte Körperbilddaten eines computergestützten, medizinischen Navigationssystems (4, 5) registriert, wobei die Markierungspunkte (6') Abbilder von Markierungen (6) an der Strahlungsquelle sind;
- eine künstliche Landmarkenanordnung (1), die im Navigationssystem (4, 5) geortet und verfolgt werden kann, wird in den Strahlengang des Fluoroskopiegerätes eingebracht, und ihre Raumposition zum Zeitpunkt der Erstellung der Fluoroskopieaufnahme wird erfasst und gespeichert, und
- die Übereinstimmung des Bildschattens (1') der künstlichen Landmarkenanordnung (1) mit der im Navigationssystem wiedergegebenen Position dieser Landmarkenanordnung (1) zum Zeitpunkt der Erstellung der Aufnahme wird mittels der Ausgabeeinheit (7) des Navigationssystems geprüft.

2. Verfahren nach Anspruch 1, bei dem, wenn eine mangelnde Übereinstimmung festgestellt wird, die Erstellung einer neuen Fluoroskopieaufnahme angefordert oder ausgelöst wird.

3. Verfahren nach einem der Ansprüche 1 oder 2, bei dem die künstliche Landmarkenanordnung wieder entfernbar in den Strahlengang des Fluoroskopiegerätes eingebracht wird.

4. Vorrichtungssystem zur Verifizierung der Registrierung eines Fluoroskopiebildes, das mit Hilfe eines Fluoroskopiegerätes erstellt wurde, mit
- Markierungen (6) an der Strahlungsquelle des Fluoroskopiegerätes, die auf einer Fluoroskopieaufnahme (2) als Markierungspunkt-Abbilder (6') abgebildet werden;
- einem computergestützten, medizinischen Navigationssystems (4, 5) mittels dem die Fluoroskopieaufnahme auf vorakquirierte Körperbilddaten registriert wird; **gekennzeichnet durch**
- eine vom Navigationssystem (4, 5) ortbare und verfolgbare, künstliche Landmarkenanordnung (1), die bei der Erstellung der Fluoroskopieaufnahme in den Strahlengang des Fluoroskopiegerätes einbringbar ist; **durch**
- eine Ausgabeeinheit (7) des Navigationssystems zur Überprüfung der Übereinstimmung des Bildschattens (1') der künstlichen Landmarkenanordnung (1) mit der im Navigationssystem wiedergegebenen Position dieser Landmarkenanordnung (1); und **durch**
- eine Speichereinrichtung, welche die Raumposition der Landmarkenanordnung (1) bei der Erstellung der Fluoroskopieaufnahme durch das Navigationssystem (4, 5) erfasst und speichert.

5. Vorrichtungssystem nach Anspruch 4, **dadurch gekennzeichnet, dass** die Speichereinrichtung ein Teil einer Computereinheit des Navigationssystems (4, 5) ist.

6. Vorrichtungssystem nach Anspruch 4 oder 5, **dadurch gekennzeichnet, dass** die künstliche Landmarkenanordnung (1) Referenzmarker (8) aufweist, die an einer Haltestruktur (9) angeordnet sind und von der Tracking- bzw. Kameraeinheit (5) des Navigationssystems positionell bestimmt und verfolgt werden können, wobei die Landmarkenanordnung insbesondere im Navigationssystem vorkalibriert bzw. der Geometrie nach bekannt ist.

7. Vorrichtungssystem nach Anspruch 6, **dadurch gekennzeichnet, dass** die Haltestruktur (9) aus einem mittels Röntgenstrahlung abbildbaren, insbesondere für Röntgenstrahlung teil- oder undurchlässigen Material besteht oder ein solches Material aufweist.

8. Vorrichtungssystem nach Anspruch 6 oder 7, **dadurch gekennzeichnet, dass** die Haltestruktur (9) kreuzförmig mit Referenzmarkem (8) an den Außenenden ausgebildet ist.

9. Programm, das, wenn es auf einem Computer läuft oder in einem Computer geladen ist, den Computer veranlasst, ein Verfahren gemäß einem der Ansprüche 1 bis 3 bis durchzuführen.

10. Computerprogramm-Speichermedium, das ein Programm nach Anspruch 9 aufweist.

## Claims

1. A method for verifying the registration of a fluoroscopy image, comprising the steps of:
- producing a fluoroscopy recording with the aid of a fluoroscopy apparatus;
- registering the fluoroscopy recording (2) onto previously acquired body image data of a computer-assisted, medical navigation system (4, 5), by means of marking points (6') mapped on it, wherein the marking points (6') are maps of markings (6) on the radiation source;
- introducing an artificial landmark array (1), which can be located and tracked in the navigation system (4, 5), into the radiation path of the fluoroscopy apparatus, and detecting and storing its spatial position at the time the fluoroscopy recording is produced; and
- checking, by means of the output unit (7) of the navigation system, the correspondence between the image shadow (1') of the artificial landmark array (1) and the position of said landmark array (1) at the time the recording was produced, as reproduced in the navigation system.

2. The method according to claim 1, wherein if an insufficient correspondence is established, a new fluoroscopy recording is requested or prompted to be produced.

3. The method according to any one of claims 1 or 2, wherein the artificial landmark array is introduced into the radiation path of the fluoroscopy apparatus such that it can be removed again.

4. A device system for verifying the registration of a fluoroscopy image produced with the aid of a fluoroscopy apparatus, comprising:
- markings (6) on the radiation source of the fluoroscopy apparatus, which are mapped on a fluoroscopy recording (2) as marking point maps (6');
- a computer-assisted, medical navigation system (4, 5), by means of which the fluoroscopy recording is registered onto previously acquired body image data;
**characterised by**:
- an artificial landmark array (1), which can be located and tracked by the navigation system (4, 5) and which can be introduced into the radiation path of the fluoroscopy apparatus at the time the fluoroscopy recording is produced; by
- an output unit (7) of the navigation system for checking the correspondence between the image shadow (1') of the artificial landmark array (1) and the position of said landmark array (1) as reproduced in the navigation system; and by
- a storage means which detects and stores the spatial position of the landmark array (1) at the time the fluoroscopy recording is produced by the navigation system (4, 5).

5. The device system according to claim 4, **characterised in that** the storage means is a part of a computer unit of the navigation system (4, 5).

6. The device system according to claim 4 or 5, **characterised in that** the artificial landmark array (1) comprises reference markers (8) which are arranged on a holding structure (9) and can be positionally determined and tracked by the tracking and/or camera unit (5) of the navigation system, wherein in particular the landmark array is pre-calibrated in the navigation system and/or known in terms of its geometry.

7. The device system according to claim 6, **characterised in that** the holding structure (9) consists of or comprises a material which can be mapped by means of x-ray radiation, in particular a material which is semi-permeable or impermeable to x-ray radiation.

8. The device system according to claim 6 or 7, **characterised in that** the holding structure (9) is formed in the shape of a cross, with reference markers (8) on the outer ends.

9. A program which, when it is running on a computer or is loaded onto a computer, causes the computer to perform a method in accordance with any one of claims 1 to 3.

10. A computer program storage medium which comprises a program according to claim 9.

## Revendications

1. Procédé pour vérifier l'enregistrement d'une image fluoroscopique présentant les étapes suivantes :
- on réalise une image fluoroscopique à l'aide d'un appareil fluoroscopique ;
- on enregistre l'image fluoroscopique (2) au moyen de points de repère (6') reproduits sur celle-ci, sur des données d'image corporelles acquises au préalable d'un système de navigation médical (4, 5) assisté par ordinateur, les points de repère (6') étant des reproductions de repères (6) sur la source de rayonnement ;
- on introduit un agencement de repères artificiels (1), qui peut être localisé et suivi dans le système de navigation (4, 5), dans le faisceau des rayons de l'appareil fluoroscopique, et on relève et mémorise sa position spatiale à l'instant de la réalisation de l'image fluoroscopique, et
- on vérifie la coïncidence de l'ombre (1') de l'agencement de repères artificiel (1) avec la position reproduite dans le système de navigation de cet agencement de repères (1) à l'instant de la réalisation de l'image au moyen de l'unité d'édition (7) du système de navigation.

2. Procédé selon la revendication 1, dans lequel on demande et déclenche la réalisation d'une nouvelle image fluoroscopique lorsque est constatée l'absence de coïncidence.

3. Procédé selon l'une des revendications 1 ou 2, dans lequel on introduit l'agencement de repères artificiels, de manière à pouvoir être à nouveau enlevé, dans le faisceau des rayons de l'appareil fluoroscopique.

4. Système de dispositif pour vérifier l'enregistrement d'une image fluoroscopique qui a été réalisé à l'aide d'un appareil fluoroscopique, comportant
- des repères (6) sur la source de rayonnement de l'appareil fluoroscopique qui sont reproduits sur une image fluoroscopique (2) en tant que reproductions de points de repère (6') ;
- un système de navigation médical (4, 5) assisté par ordinateur au moyen duquel l'image fluoroscopique est enregistrée sur des données d'image corporelles acquises au préalable ; **caractérisé par**
- un agencement de repères artificiels (1) qui peut être localisé et suivi par le système de navigation (4, 5) et qui peut être introduit dans le faisceau des rayons de l'appareil fluoroscopique lors de la réalisation de l'image fluoroscopique ; par
- une unité d'édition (7) du système de navigation pour la vérification de la coïncidence de l'ombre (1') de l'agencement de repères artificiels (1) avec la position reproduite dans le système de navigation de cet agencement de repères (1) ; et par
- un dispositif à mémoire qui relève et mémorise la position spatiale de l'agencement de repères (1) lors de la réalisation de l'image fluoroscopique par le système de navigation (4, 5).

5. Système de dispositif selon la revendication 4, **caractérisé en ce que** le dispositif à mémoire fait partie d'une unité informatique du système de navigation (4, 5).

6. Système de dispositif selon la revendication 4 ou 5, **caractérisé en ce que** l'agencement de repères artificiels (1) comporte des repères de référence (8) qui sont disposés sur une structure de support (9) et qui peuvent être déterminés et suivis dans leur position par l'unité de poursuite ou de caméra (5) du système de navigation, l'agencement de repères étant pré-étalonné en particulier dans le système de navigation ou sa géométrie étant connue.

7. Système de dispositif selon la revendication 6, **caractérisé en ce que** la structure du support (9) est constituée d'un matériau qui peut être reproduit au moyen de rayons X, en partie perméable ou imperméable en particulier aux rayons X, ou comporte un matériau de ce type.

8. Système de dispositif selon la revendication 6 ou 7, **caractérisé en ce que** la structure du support (9) est réalisée en forme de croix avec des repères de référence (8) aux extrémités extérieures.

9. Programme qui lorsqu'il tourne sur un ordinateur ou est chargé dans un ordinateur, amène l'ordinateur à exécuter un procédé selon l'une des revendications 1 à 3.

10. Support de mémoire de programme d'ordinateur qui comporte un programme selon la revendication 9.
